# EUROPEAN PATENT SPECIFICATION

(11) **EP 0 891 378 B1**
(45) Date of publication and mention of the grant of the patent: **13.11.2002**
(21) Application number: 97905000.2
(22) Date of filing: 27.02.1997
(51) Int. Cl.: C07K 14/605, A61K 38/26

(54) **USE OF A PHARMACEUTICAL COMPOSITION COMPRISING AN APPETITE-SUPPRESSING PEPTIDE**
Gebrauch einer pharmazeutischen Zusammensetzung, enthaltend ein appetitzügelndes Peptid
UTILISATION D'UNE COMPOSITION PHARMACEUTIQUE CONTENANT UN PEPTIDE VISANT A REDUIRE L'APPETIT

(30) Priority: 01.03.1996 DK 23096; 01.03.1996 DK 23196
(43) Date of publication of application: 20.01.1999
(60) Divisional application: 01122701.4
(73) Proprietor: NOVO NORDISK A/S, 2880 Bagsvaerd (DK)
(72) Inventor: THIM, Lars, DK-2820 Gentofte (DK); WULFF, Birgitte, Schjellerup, DK-2830 Virum (DK); JUDGE, Martin, Edward, DK-1363 Kobenhavn K (DK); MADSEN, Ole, Dragsbak, DK-2860 Soborg (DK); HOLST, Jens, Juul, DK-2900 Hellerup (DK)
(74) Representative: Stephensen, Birgitte Borregaard
(86) International application number: DK9700086
(87) International publication number: WO97031943

(56) References cited:
- WO-A-93/18786
- WO-A-96/32414
- NATURE, Volume 379, January 1996, M.D. TURTON et al., "A Role for Glucagon-Like Peptide-1 in the Central Regulation of Feeding", page 69.
- DIABETIC MEDICINE, Volume 12, 1995, J. RACHMAN et al., "Drugs on the Horizon for Treatment of Type 2 Diabetes", pages 467-478.

## Description

The present invention relates to use of a pharmaceutical composition comprising an appetite-suppressing peptide or an appetite-suppressing peptide-containing fraction, as well as a method of obtaining appetite regulation by means of said peptide.

### BACKGROUND OF THE INVENTION

Glucagon is produced by the pancreatic A-cell and released in response to low blood glucose levels. Its major site of action is the liver where it stimulates glucose production. It is thus the major hormone counteracting insulin in blood glucose homeostasis (Unger, R. H. and L. Orci (1990). Glucagon, in: Diabetes Mellitus, 4th ed. New York, Elsevier. pp 104-120).

Glucagon is processed from a larger precursor by limited proteolysis.
Molecular cloning of the glucagon gene revealed that the proglucagon precursor contained not only glucagon but also two additional glucagon-like peptides named GLP-1 and GLP-2. GLP-1 and GLP-2 are encoded by separate exons suggesting distinct biological activities. It was later demonstrated that the proglucagon precursor was subjected to differential processing in the three different tissues known to produce proglucagon: the pancreatic A-cell, the intestinal L-cell, and in the central nervous system (CNS). Glucagon is thus selectively excised from the precursor in the islet A-cell, while GLP-1 and GLP-2 are selectively liberated from the intestinal L-cell and the CNS [reviewed in (Unger, R. H. and L. Orci (1990). Glucagon. in: Diabetes Mellitus, 4th ed. New York, Elsevier. pp 104-120)].

Specific GLP-1 receptors have been identified (Thorens, B. (1992) Proc.Natl. Acad. Sci. USA **89:** 8641-8645) which are clearly distinct from the glucagon receptor (L. J Jelinek, et al. (1993) Science **259:** 1614-1616) and they have different tissue distributions (R. V Campos, et al. (1994) Endocrinology **134:** 2156-2164). GLP-1 is released from the L-cell after a meal and functions as an incretin hormone (i.e. it potentiates glucose induced insulin release from the pancreatic B-cell). The GLP-1 receptor is thus expressed at high levels on the surface of islet B-cells (K. Moens, et al. (1996) Diabetes **45:** 257-261).

Induction of intestinal epithelial proliferation by GLP-2 was demonstrated (Drucker, D. J. et al (1996) Proc. Natl. Acad. Sci. USA **93**: 7911-7916) and treatment of gastrointestinal deseases by cells grown in GLP-2 medium was disclosed (Drucker, D.J and Keneford, J.R., WO 96/32414). No GLP-2 receptor has so far been reported.

### Proglucagon derived peptides and feeding behaviour

We have previously reported the derivation and establishment of transplantable anorectic glucagonomas (O. D. Madsen, et al. (1993) Endocrinology **133**: 2022-2030) as well as of hypoglycemic insulinomas in the rat (O. D Madsen, et al. (1988) Proc.Natl. Acad. Sci. USA **85:** 6652-6656). Such tumors can be derived from common clonal origin of pluripotent MSL-cells (O. D .Madsen, et al. (1986) J. Cell Biol. **103**: 2025-2034) and reflects a maturation process towards islet A-cell and B-cells, respectively (O. D. Madsen, et al. (1993) Endocrinology 133: 2022-2030).

The glucagonoma associated anorexia is very severe: it has an acute onset and leads after few days to a complete stop in food intake. This severity of anorexia is hardly matched by other experimental tumors in rodents and suggests the production by the glucagonoma of a very powerful satiety factor which acts by a peripheral route of administration. It has previously been demonstrated that the anorectic glucagonomas displayed an unphysiological processing resulting in the formation of both glucagon and GLP-1 (O. D. Madsen, et al. (1993) Endocrinology **133:** 2022-2030). Moreover, a non-anorectic glucagonoma variant was unable to process the precursor (O. D. Madsen, et al. (1995) Scand. J. Clin, Lab. Invest.**55,** suppl **220:** 27-36. Weight loss is mentioned as a component also of the glucagonoma syndrome in man (J. J. Hoist (1985) Glucagon-producing tumors, in: Hormone-producing tumors of the gastrointestinal tract. New York, Churchill Livingstone. pp **57-84**) although with a high degree of variability among different patients (S. J. Bhathena, et al. (1981). Glucagonoma and glucagonoma syndrome, in: Glucagon. Physiology, pathophysiology and morphology of the pancreatic A-cells. New York, Elsevier. 413-438).

### Glucagon

Glucagon has been shown to be involved in the regulation of spontaneous meal size in rats but the overall effect is minimal and is exerted via the vagal connections to the liver (N. Geary, et al. (1993) Am. J. Physiol. **264:** R116-R122). This effect is observed only by hepatic portal infusion of glucagon while intraperitoneal administration of pharmacological doses show no effect on food intake in fasted rats (O. D. Madsen, et al. (1993) Endocrinology **133:** 2022-2030).

### GLP-1

A central role for GLP-1 in the regulation of feeding was recently reported (M. D. Turton, et al. (1996) Nature **379:** 69-72). Intracerebroventricular (ICV) administration of GLP-1 inhibited feeding in fasted rats. Again peripheral administration of GLP-1 had no effect on feeding behaviour (M. D. Turton, et at. (1996) Nature **379:** 69-72; O. D. Madsen, et al. (1993) Endocrinology **133:** 2022-2030) suggesting that tumor produced GLP-1 may not contribute significantly to the observed anorexia.

### SUMMARY OF THE INVENTION

It has been found that GLP-2 has a powerful effect on inhibiting food intake when administered peripherally.
It is proposed that GLP-2 normally released together with GLP-1 from the intestinal L-cell serves its own distinct role as a peripheral satiety factor.

The object of the invention is defined in the claims.

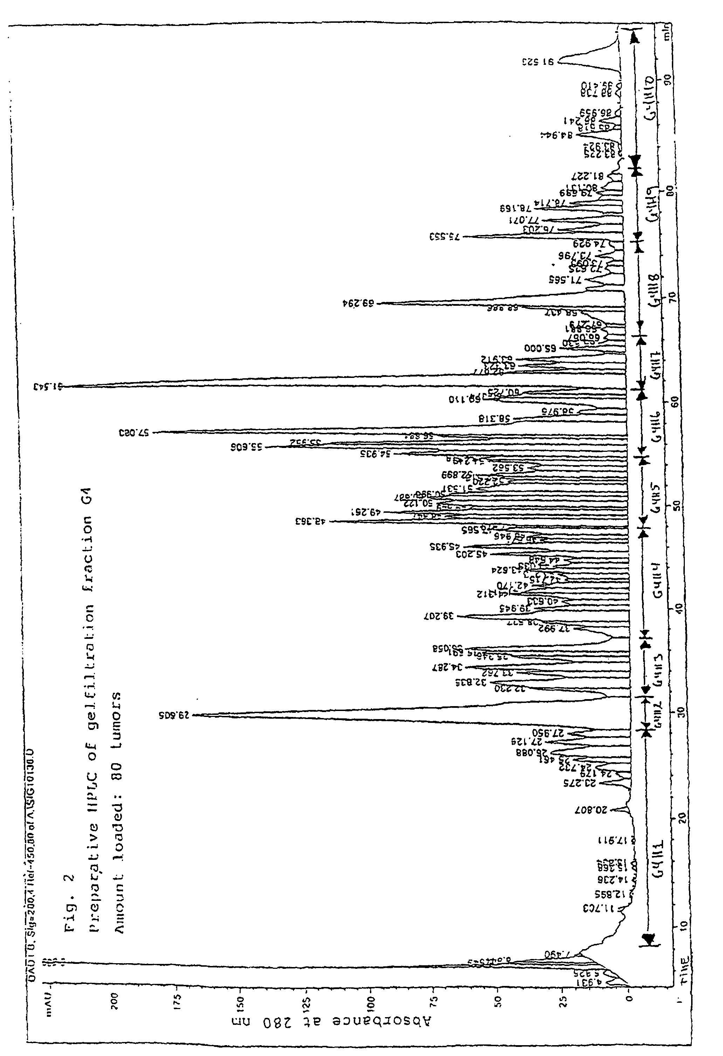

Accordingly, the present invention relates to use of a pharmaceutical composition comprising, together with a pharmaceutically acceptable excipient or vehicle, an HPLC fraction of a glucagonoma tumour extract prepared by acid ethanol extraction, gel filtration and preparative HPLC, said fraction being shown as fraction G4H9 in Fig. 2 and containing glucagon-like peptide 2 (GLP-2) as a major component (i.e. more than 40%) or comprising any single component of said fraction or a combination of two or more of the components of said fraction.

In another aspect, the invention relates to use of a pharmaceutical composition comprising glucagon-like peptide-2 (GLP-2) or a variant or homologue thereof for the prohylaxis or treatment of diseases or disorders associated with impaired appetite regulation.

In a further aspect, the invention relates to use of a pharmaceutical composition comprising a peptide with the following amino acid sequence 
X¹ H X² D G S F S D E M N T X³ L D X⁴ L A X⁵ X⁶ D F I N W L X⁷ X⁸ T K I T D
X⁹
 wherein
X¹ is NH₂, DFPEEVAIVEELGRR, DPPEEVTIVEELGRR, DFPEEVNIVEELRRR, or a fragment thereof,
X² is Gly,
X³ is IIe or Val,
X⁴ is Asn, Ser or His,
X⁵ is Ala or Thr,
X⁶ is Arg or Lys,
X⁷ is Ile or Leu,
X⁸ is Gln or His, or
X⁹ is OH, Lys, Arg, Arg-Lys, Lys-Arg, Arg-Arg or Lys-Lys
for the prohylaxis or treatment of diseases or disorders associated with impaired appetite regulation.

In a still further aspect, the invention relates to the use of a peptide as specified herein for the preparation of a medicament for a method of treating diseases or disorders associated with impaired appetite regulation, the method comprising administering to an individual in need of such treatment an amount of a peptide as specified herein sufficient to suppress appetite or induce satiety in said individual.

In a still further aspect, the invention relates to the use of a peptide as specified herein for the manufacture of a medicament for the prophylaxis or treatment of diseases or disorders associated with impaired appetite regulation.

### DETAILED DESCRIPTION OF THE INVENTION

In the present description, the term "peptide" is understood to include the mature GLP-2 peptide or a precursor form thereof as well a functional fragment thereof which essentially has the activity of the full-length peptide. Furthermore, the term "peptide" is intended to include homologues of said peptide. Such homologues comprise an amino acid sequence exhibiting a degree of identity of at least 50 %, such as at least 75 %, and more particularly at least 90% identity with the amino acid sequence of human GLP-2. The degree of identity may be determined by conventional methods, see for instance, Altshul et al., Bull. Math. Bio. 48: 603-616, 1986, and Henikoff and Henikoff, Proc. Natl. Acad. Sci. USA 89: 10915-10919, 1992.

Homologues of the present peptide may have one or more amino acid substitutions, deletions or additions. These changes may be of a minor nature, that is conservative amino acid substitutions that do not significantly affect the folding or activity of the peptide, small deletions, typically of one to about five amino acids, small amino- or carboyxyl-terminal extensions, such as an amino-terminal methionine residue, a small linker peptide of up to about 15 residues, or a small extension that facilitates purification, such as a polyhistidine tract, an antigenic epitope or a binding domain. See in general Ford et al., Protein Expression and Purification 2: 95-107, 1991. Examples of conservative substitutions are within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine).

The homologue may be an allelic variant, i.e. an alternative form of a gene that arises through mutation, or an altered peptide encoded by the mutated gene, but having substantially the same activity as the native GLP-2 peptide. Hence mutations can be silent (no change in the encoded peptide) or may encode peptides having altered amino acid sequence.

The homologue of the present peptide may also be a species homologue, i.e. a peptide with a similar activity derived from another species. Examples of species homologues of the GLP-2 peptide are human, bovine, rat, hamster, guinea pig and porcine GLP-2.

In a preferred embodiment of the present invention, the GLP-2 peptide is one in which X¹ is NH₂, X² is Ala, X³ is Ile, X⁴ is Asn, X⁵ is Ala, X⁶ is Arg, X⁷ is Ile, X⁸ is Gln, or X⁹ is OH. In particular, the peptide has the following amino acid sequence

H A D G S F S D E M N T I L D N L A A R D F I N W L I Q T K I T D
(human GLP-2) or
H A D G S F S D E M N T I L D N L A T R D F I N W L I Q T K I T D
(rat GLP-2) or
H A D G S F S D E M N T V L D N L A T R D F I N W L L H T K I T D
(porcine GLP-2).

A homologue of the peptide may be isolated by preparing a genomic or cDNA library of a cell of the species in question, and screening for DNA sequences coding for all or part of the homologue by using synthetic oligonucleotide probes in accordance with standard techniques, e.g. as described by Sambrook et al., Molecular Cloning:A Laboratory Manual, 2nd. Ed. Cold Spring Harbor Laboratory, Cold Spring Harbor, New York, 1989, or by means of polymerase chain reaction (PCR) using specific primers as described by Sambrook et al., supra.

The present invention also relates to a composition comprising a variant of the GLP-2 peptide. The variant is one in which one or more amino acid residues have been substituted by other amino acid residues. In a particularly preferred embodiment Ala has been substituted by Gly in position 2 of the mature peptide. It is expected that this variant will exhibit a longer plasma half-life than the native peptide, which is an advantage because the dosage required to obtain an adequate appetite-suppressing or satiety-inducing effect will generally be smaller.

The GLP-2 peptide or homologue or variant thereof as specified above may be made by recombinant DNA techniques in accordance with procedures well established in the art.

More specifically, a DNA sequence encoding the GLP-2 peptide may be isolated or synthesized on the basis of the published human preproglucagon DNA sequence (cf. J.W. White et al., *Nucleic Acids Res. 14*, 1986, pp. 4719-4730; G.I. Bell et al., *Nature 304,* 1983, pp. 368-371), for instance obtained by preparing a genomic or cDNA library from an appropriate tissue and screening for DNA sequences coding for all or part of the GLP-2 peptide by hybridization using synthetic oligonucleotide probes in accordance with standard techniques (cf. Sambrook et al., supra). For the present purpose, the DNA sequence encoding the GLP-2 peptide is preferably of human origin.

The DNA construct encoding the GLP-2 peptide may also be prepared synthetically by established standard methods, e.g. the phosphoamidite method described by Beaucage and Caruthers, Tetrahedron Letters 22 (1981), 1859-1869, or the method described by Matthes et al., EMBO Journal 3 (1984), 801-805. According to the phosphoamidite method, oligonucleotides are synthesized, e.g. in an automatic DNA synthesizer, purified, annealed, ligated and cloned in suitable vectors.

Furthermore, the DNA construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA construct, in accordance with standard techniques.

The DNA construct may also be prepared by polymerase chain reaction using specific primers, for instance as described in US 4,683,202 or Saiki et al., Science 239 (1988), 487 - 491, or Sambrook et al., *supra.*

In a currently preferred embodiment, the DNA construct comprises the DNA sequence shown in Fig. 3 of G.I. Bell et al., *Nature 304,* 1983, pp. 368-371, as well as nucleic acid sequences encoding human GLP-2, but which differ from the DNA sequence shown in Fig. 3 of Bell et al., *supra,* by virtue of the degeneracy of the genetic code. The DNA construct further includes nucleic acid sequences which hybridize to a nucleic acid molecule (either genomic, synthetic or cDNA or RNA) encoding human GLP-2 under the conditions of high stringency (i.e. presoaking in 5X SSC and prehydbridizing for 1 hr. at about 40°C in a solution of 20% formamide, 5X Denhardt's solution, 50 mM sodium phosphate, pH 6.8, and 50 µg denatured sonicated calf thymus DNA, followed by hybridization in the same solution supplemented with 100 µM ATP for 18 hrs. at about 40°C, followed by a wash in 0.4X SSC at a temperature of about 45°C). This could, for instance, be DNA sequences encoding GLP-2 from other species, e.g. rat, bovine, hamster, guinea pig or porcine GLP-2.

To express GLP-2, the DNA construct encoding the GLP-2 peptide is inserted into an appropriate recombinant vector. This may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the GLP-2 peptide is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the peptide.

The promoter may be any DNA sequence which shows transcriptional activity in the host cell of choice and may be derived from genes encoding proteins either homologous or heterologous to the host cell.

Examples of suitable promoters for directing the transcription of the DNA encoding the GLP-2 peptide in mammalian cells are the SV40 promoter (Subramani et al., Mol. Cell Biol. 1 (1981), 854-864), the MT-1 (metallothionein gene) promoter (Palmiter et al., Science 222 (1983), 809 - 814) or the adenovirus 2 major late promoter.

An example of a suitable promoter for use in insect cells is the polyhedrin promoter (US 4,745,051; Vasuvedan et al., FEBS Lett. 311, (1992) 7 - 11), the P10 promoter (J.M. Vlak et al., J. Gen. Virology 69, 1988, pp. 765-776), the *Autographa californica* polyhedrosis virus basic protein promoter (EP 397 485), the baculovirus immediate early gene 1 promoter (US 5,155,037; US 5,162,222), or the baculovirus 39K delayed-early gene promoter (US 5,155,037; US 5,162,222).

Examples of suitable promoters for use in yeast host cells include promoters from yeast glycolytic genes (Hitzeman et al., J. Biol. Chem. 255 (1980), 12073 - 12080; Alber and Kawasaki, J. Mol. Appl. Gen. 1 (1982), 419 - 434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., Nature 304 (1983), 652 - 654) promoters.

Examples of suitable promoters for use in filamentous fungus host cells are, for instance, the ADH3 promoter (McKnight et al., The EMBO J. 4 (1985), 2093 - 2099) or the tpiA promoter. Examples of other useful promoters are those derived from the gene encoding *A. oryzae* TAKA amylase, *Rhizomucor miehei* aspartic proteinase, *A*. *niger* neutral α-amylase, *A. niger* acid stable α-amylase, *A. niger* or *A. awamori* glucoamylase (gluA), *Rhizomucor miehei* lipase, *A. oryzae* alkaline protease, *A. oryzae* triose phosphate isomerase or *A. nidulans* acetamidase. Preferred are the TAKA-amylase and gluA promoters.

Examples of suitable promoters for use in bacterial host cells include the promoter of the *Bacillus stearothermophilus* maltogenic amylase gene, the *Bacillus licheniformis* alpha-amylase gene, the *Bacillus amyloliquefaciens* BAN amylase gene, the *Bacillus subtilis* alkaline protease gen, or the *Bacillus pumilus* xylosidase gene, or by the phage Lambda P_{R} or P_{L} promoters or the E. coli lac, trp or tac promoters.

The DNA sequence encoding the GLP-2 peptide may also, if necessary, be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) terminators. The vector may further comprise elements such as polyadenylation signals (e.g. from SV40 or the adenovirus 5 Elb region), transcriptional enhancer sequences (e.g. the SV40 enhancer) and translational enhancer sequences (e.g. the ones encoding adenovirus VA RNAs).

The recombinant vector may further comprise a DNA sequence enabling the vector to replicate in the host cell in question. An example of such a sequence (when the host cell is a mammalian cell) is the SV40 origin of replication.

When the host cell is a yeast cell, suitable sequences enabling the vector to replicate are the yeast plasmid 2µ replication genes REP 1-3 and origin of replication.

The vector may also comprise a selectable marker, e.g. a gene the product of which complements a defect in the host cell, such as the gene coding for dihydrofolate reductase (DHFR) or the *Schizosaccharomyces pombe* TPI gene (described by P.R. Russell, Gene 40, 1985, pp. 125-130), or one which confers resistance to a drug, e.g. ampicillin, kanamycin, tetracyclin, chloramphenicol, neomycin, hygromycin or methotrexate. For filamentous fungi, selectable markers include amdS, pyrG, argB, niaD, sC.

To direct the GLP-2 peptide into the secretory pathway of the host cells, a secretory signal sequence (also known as a leader sequence, preprosequence or presequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

For secretion from yeast cells, the secretory signal sequence may encode any signal peptide which ensures efficient direction of the expressed peptide into the secretory pathway of the cell. The signal peptide may be naturally occurring signal peptide, or a functional part thereof, or it may be a synthetic peptide. Suitable signal peptides have been found to be the α-factor signal peptide (cf. US 4,870,008), the signal peptide of mouse salivary amylase (cf. O. Hagenbuchle et al., Nature 289, 1981, pp. 643-646), a modified carboxypeptidase signal peptide (cf. L.A. Valls et al., Cell 48, 1987, pp. 887-897), the yeast BAR1 signal peptide (cf. WO 87/02670), or the yeast aspartic protease 3 (YAP3) signal peptide (cf. M. Egel-Mitani et al., Yeast 6, 1990, pp. 127-137).

For efficient secretion in yeast, a sequence encoding a leader peptide may also be inserted downstream of the signal sequence and uptream of the DNA sequence encoding the GLP-2 peptide. The function of the leader peptide is to allow the expressed peptide to be directed from the endoplasmic reticulum to the Golgi apparatus and further to a secretory vesicle for secretion into the culture medium (i.e. export of the peptide across the cell wall or at least through the cellular membrane into the periplasmic space of the yeast cell). The leader peptide may be the yeast α-factor leader (the use of which is described in e.g. US 4,546,082, EP 16 201, EP 123 294, EP 123 544 and EP 163 529). Alternatively, the leader peptide may be a synthetic leader peptide, which is to say a leader peptide not found in nature. Synthetic leader peptides may, for instance, be constructed as described in WO 89/02463 or WO 92/11378.

For use in filamentous fungi, the signal peptide may conveniently be derived from a gene encoding an *Aspergillus* sp. amylase or glucoamylase, a gene encoding a *Rhizomucor miehei* lipase or protease, a *Humicola lanuginosa* lipase. The signal peptide is preferably derived from a gene encoding A. *oryzae* TAKA amylase, *A. niger* neutral α-amylase, *A. niger* acid-stable amylase, or *A. niger* glucoamylase.

For use in insect cells, the signal peptide may conveniently be derived from an insect gene (cf. WO 90/05783), such as the lepidopteran *Manduca sexta* adipokinetic hormone precursor signal peptide (cf. US 5,023,328).

The procedures used to ligate the DNA sequences coding for the GLP-2 peptide, the promoter and optionally the terminator and/or secretory signal sequence, respectively, and to insert them into suitable vectors containing the information necessary for replication, are well known to persons skilled in the art (cf., for instance, Sambrook et al., op.cit.).

The DNA sequence encoding the GLP-2 peptide introduced into the host cell may be either homologous or heterologous to the host in question. If homologous to the host cell, i.e. produced by the host cell in nature, it will typically be operably connected to another promoter sequence or, if applicable, another secretory signal sequence and/or terminator sequence than in its natural environment. The term "homologous" is intended to include a cDNA sequence encoding a polypeptide native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell which is capable of producing the present peptide and includes bacteria, yeast, fungi and higher eukaryotic cells.

Examples of bacterial host cells which, on cultivation, are capable of producing the GLP-2 peptide are grampositive bacteria such as strains of *Bacillus,* such as strains of *B. subtilis, B. licheniformis, B. lentus, B. brevis, B. stearothermophilus, B. alkalophilus, B. amyloliquefaciens, B. coagulans, B. circulans, B. lautus, B. megatherium* or *B. thuringiensis,* or strains of *Streptomyces,* such as *S. lividans* or *S. murinus,* or gramnegative bacteria such as *Echerichia coli.* The transformation of the bacteria may be effected by protoplast transformation or by using competent cells in a manner known per se (cf. Sambrook et al., supra).

When expressing the peptide in bacteria such as *E. coli*, the peptide may be retained in the cytoplasm, typically as insoluble granules (known as inclusion bodies), or may be directed to the periplasmic space by a bacterial secretion sequence. In the former case, the cells are lysed and the granules are recovered and denatured after which the peptide is refolded by diluting the denaturing agent. In the latter case, the peptide may be recovered from the periplasmic space by disrupting the cells, e.g. by sonication or osmotic shock, to release the contents of the periplasmic space and recovering the peptide.

Examples of suitable mammalian cell lines are the COS (ATCC CRL 1650), BHK (ATCC CRL 1632, ATCC CCL 10), CHL (ATCC CCL39) or CHO (ATCC CCL 61) cell lines. Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet. 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841 - 845.

Examples of suitable yeasts cells include cells of *Saccharomyces* spp. or *Schizosaccharomyces* spp., in particular strains of *Saccharomyces cerevisiae* or *Saccharomyces kluyveri.* Methods for transforming yeast cells with heterologous DNA and producing heterologous polypeptides therefrom are described, e.g. in US 4,599,311, US 4,931,373, US 4,870,008, 5,037,743, and US 4,845,075, all of which are hereby incorporated by reference. Transformed cells are selected by a phenotype determined by a selectable marker, commonly drug resistance or the ability to grow in the absence of a particular nutrient, e.g. leucine. A preferred vector for use in yeast is the POT1 vector disclosed in US 4,931,373. The DNA sequence encoding the GLP-2 peptide may be preceded by a signal sequence and optionally a leader sequence, e.g. as described above. Further examples of suitable yeast cells are strains of *Kluyveromyces*, such as K. *lactis, Hansenula,* e.g. *H. polymorpha,* or *Pichia,* e.g. *P. pastoris* (cf. Gleeson et al., J. Gen. Microbiol. 132, 1986, pp. 3459-3465; US 4,882,279).

Examples of other fungal cells are cells of filamentous fungi, e.g. *Aspergillus* spp., *Neurospora* spp., *Fusarium* spp. or *Trichoderma* spp., in particular strains of *A. oryzae, A. nidulans* or *A. niger.* The use of *Aspergillus* spp. for the expression of proteins is described in, e.g., EP 272 277 and EP 230 023. The transformation of *F. oxysporum* may, for instance, be carried out as described by Malardier et al., 1989, Gene 78: 147-156.

When a filamentous fungus is used as the host cell, it may be transformed with the DNA construct encoding the GLP-2 peptide, conveniently by integrating the DNA construct in the host chromosome to obtain a recombinant host cell. This integration is generally considered to be an advantage as the DNA sequence is more likely to be stably maintained in the cell. Integration of the DNA constructs into the host chromosome may be performed according to conventional methods, e.g. by homologous or heterologous recombination.

Transformation of insect cells and production of heterologous polypeptides therein may be performed as described in US 4,745,051; US 4,879,236; US 5,155,037; 5,162,222; EP 397,485) all of which are incorporated herein by reference. The insect cell line used as the host may suitably be *a Lepidoptera* cell line, such as *Spodoptera frugiperda* cells or *Trichoplusia ni* cells (cf. US 5,077,214). Culture conditions may suitably be as described in, for instance, WO 89/01029 or WO 89/01028, or any of the aforementioned references.

The transformed or transfected host cell described above is then cultured in a suitable nutrient medium under conditions permitting the expression of the GLP-2 peptide, after which the resulting GLP-2 peptide is recovered from the culture.

The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The GLP-2 peptide produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, gelfiltration chromatography, affinity chromatography, or the like.

In the pharmaceutical composition of the invention, the GLP-2 peptide may be formulated by any of the established methods of formulating pharmaceutical compositions, e.g. as described in Remington's Pharmaceutical Sciences, 1985. The composition may be in a form suited for systemic injection or infusion and may, as such, be formulated with a suitable liquid vehicle such as sterile water or an isotonic saline or glucose solution. The compositions may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc.

The pharmaceutical composition of the present invention may also be adapted for nasal, transdermal, pulmonal or rectal administration. The pharmaceutically acceptable carrier or diluent employed in the composition may be any conventional solid carrier. Examples of solid carriers are lactose, terra alba, sucrose, talc, gelatin, agar, pectin, acacia, magnesium stearate and stearic acid. Similarly, the carrier or diluent may include any sustained release material known in the art, such as glyceryl monostearate or glyceryl distearate, alone or mixed with a wax.

It may be of particular advantage to provide the composition of the invention in the form of a sustained release formulation. As such, the composition may be formulated as microcapsules or microparticles containing the GLP-2 peptide encapsulated by or dispersed in a suitable pharmaceutically acceptable biodegradable polymer such as polylactic acid, polyglycolic acid or a lactic acid/glycolic acid copolymer.

For nasal administration, the preparation may contain GLP-2 peptide dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

Generally, the compounds of the present invention are dispensed in unit dosage form comprising 0.5-500 mg of the peptide together with a pharmaceutically acceptable carrier per unit dosage.

The GLP-2 peptide is contemplated to be advantageous to use in appetite suppression or satiety induction, such as for the prophylaxis or treatment of diseases or disorders associated with impaired appetite regulation. Examples of such diseases or disorders are obesity and type II diabetes. The dosage of the GLP-2 peptide administered to a patient will vary with the type and severity of the condition to be treated, but is generally in the range of from about 10 µg/kg to about 5 mg/kg body weight.

In the pharmaceutical composition of the invention, the GLP-2 peptide may be combined with another appetite-suppressing or satiety-inducing agent. An example of such an agent is GLP-1 which has been shown to have some effect on appetite suppression (cf. M.D. Turton et al., *Nature 379,* 4 January 1996, pp. 69-72).

It is further contemplated that the GLP-2 peptide in suitably labelled form, e.g. radiolabelled GLP-2, may be used to identify a receptor for GLP-2 in binding studies using tissue(s) expected to express the GLP-2 receptor, e.g. hypothalamus tissue. Once localized by GLP-2 binding, the receptor may be cloned by expression cloning, i.e. by preparing a cDNA library of the tissue in question, cloning the cDNA Into suitable vectors and introducing the vectors into an appropriate cell to effect expression of the cDNA, after which a clone expressing the receptor is identified by binding to GLP-2. A cell line stably expressing the receptor may then be used in a screening assay for GLP-2 agonists (i.e. compounds acting on the receptor to induce satiety or suppress appetite) or GLP-2 antagonists (i.e. compounds which antagonize the action of GLP-2 on the receptor, e.g. for use in the treatment of cancer anorexia or anorexia nervosa).

The invention is further illustrated in the following examples which are not in any way intended to limit the scope of the invention as claimed.

### Example 1

### Acid ethanol extraction of tumor tissue.

Anorectic tumors were produced in rats as previously described (Madsen, O.D. et al. (1993) Endocrinology 133, 2022-2030). Fifty anorectic 12C3AN (MSL-G-AN) tumours (at -80°C) corresponding to 50.07 g of wet tissue were homogenised at 4°C with 700 ml of acid ethanol (96% ethanol/0.7M HCl, 3/1, vol/vol). The homogenisation was carried out for 5 min in a precooled (4°C) 2 liter Waring Commercial Blender at maximum speed. After homogenisation the mixture was stirred at 4°C for 16 hours. The mixture was centrifuged at 9000 RPM at 4°C for 1 hour. The volume of the supernatant was reduced to 20% by vacuum rotation. During this process, in which the main part of the ethanol is removed, some precipitate is formed. This precipitate was removed by centrifugation at 4°C for one hour at 20.000 RPM. The supernatant, which still contained some lipid-like material, was filtered and applied to a LiChroprep RP-18 (Merck) column (2.5 × 10cm) equilibrated with 0.1% TFA at a flow rate of 2 ml/min. The column was washed with 100 ml of 0.1% of TFA at a flow rate of 4 ml/min. Bound material was eluted with 400 ml of 0.1% TFA containing 70% (vol/vol) of acetonitrile. The acetonitrile was removed by vacuum rotation and the resulting mixture was lyophilised. After lyophilization the material was dissolved in 50 ml of water and the pH was adjusted to 5.3 with 425 µl of 1N NaOH. Further titration of the mixture to pH 6.0 resulted in the formation of a precipitate. Upon back titration to pH 5.3 this precipitate was dissolved again. Therefore the pH was left at 5.3 and the mixture was lyophilised.
The total yield of lyophilised material from 50 tumours was 359 mg of dry powder.

### Example 2

### First purification step: gel filtration on Sephadex G-75

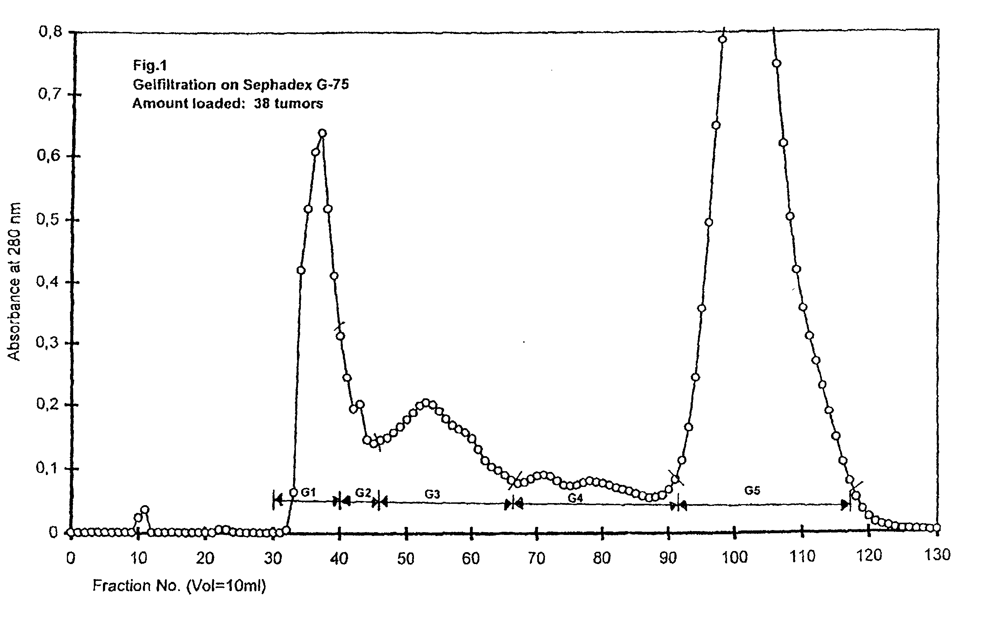

Lyophilized material (278 mg) from the acid ethanol extract corresponding to **38** individual tumors was redissolved in 20 ml of 1 M HAc and applied to Sephadex G75 column (5 x 50 cm). The column was equilibrated and eluted with 1 M HAc at a flow rate of 55 ml/h, and fractions corresponding to 10 ml were collected. The absorption at 280 nm was recorded for each fraction. The gel filtration chromatogram is shown in Fig. 1. Individual fractions were pooled in the following 5 main fractions: G1 (Fr. 30-39), G2 (Fr. 40-45), G3 (Fr. 46-66), G4 (Fr. 67-91) and G5 (Fr. 92-118) and subjected to bioassay after lyophilization.

### Example 3

### Second purification step: Preparative HPLC of the G4 pool

Some of the appetite suppression activity of gel filtration pools showed the activity to be present in the G4 pool, and this pool was further fractionated by preparative HPLC. Lyophilized G4 material (corresponding to 80 tumors) was redissolved in 15 ml 0.1% TFA and pumped onto a Vydac 214TP1022 C4 column (2.2 x 25 cm) equilibrated in 0.1% TFA. The column was washed with 20 ml of 0.1% TFA, followed by 100 ml of MeCN/H₂O/TFA (10.0:89.9:0.1, v/v/v). The material was eluted at 25°C at a flow rate of 4 ml/min with a linear gradient formed from MeCN/H₂O/TFA (10:79.9:0.1 v/v/v) and MeCN/H₂O/TFA (65.0:34.9:0.1, v/v/v) over 110 min. UV absorption was monitored at 214 nm and 280 nm. The HPLC chromatogram (monitored at 280nm) is shown in Fig. 2. Fractions corresponding to 10 main pools were generated as indicated in Fig. 2. The volume was reduced to approx. 25% by vacuum rotation and the fractions were lyophilized and tested in the bioassay.
The appetite suppression activity was found in fraction G4H9 (Example 6) and the peptides of this fraction were analysed by amino acid sequence analysis and mass spectrometry analysis (Example 4).

### Example 4

### Chemical characterisation of the peptides in fraction G4H9

Amino acid sequence analysis was carried out by automated Edman degradation using an Applied Biosystems Model 477 gas-phase sequencer essentially as described by the manufacturer. Mass spectrometry analysis was performed using an API III LC/MS/MS system (Sciex, Thornhill, Ont., Canada). The triple quadrupole instrument has a mass-to-charge (m/z) range of 2400 and is fitted with a pneumatically assisted electrospray (also referred to as ion-spray) interface (Bruins, A.P., Covey, T.R., & Henion, J.D. (1987) *Anal. Chem. 59,* 2642-2646 and Covey, T.R., Bonner, R.F., Shushan, B.I., & Henion, J.D. (1988) *Rapid Commun. Mass Spectrom. 2,* 249-256). Sample introduction was done by a syringe infusion pump (Sage Instruments, Cambridge, MA) through a fused capillary /75 mm i.d.) with a liquid flow rate set at 0.5-1 ml/min. The instrument m/z scale was calibrated with the singly-charged ammonium adduct ions of poly(propylene glycols) (PPGs) under unit resolution. The accuracy of mass measurements is generally better than 0.02%.

### Fraction G4H9:

The dominating peptide in this fraction was found to have the following amino acid sequence: H A D G S F S D E M N T I L D N L A T R D F I N W L I Q T K I T D The molecular weight found by mass spectrometry was: 3796.
This peptide is identical to rat GLP-2 (1-33). Minor amounts of the following two peptides were also found: 
D F P E E V A I A E E L G R R H A D G S F S D E M N T I L D N L A T R D F I N
W L I Q T K I T D and
H D E F E R H A E G T F T S D V S S Y L E G Q A A K E F I A W L V K G R
 These peptides are identical to rat GLP-2 which is N-terminally extended with spacer peptide 2 and rat GLP-1 (1-36amide), respectively.

### Example 5

### Test method for measuring appetite suppression in mice.

Mice were deprived of their normal feed for two days and given free access to a 20% sucrose solution on the first day of feed deprivation. After the 2-day feed deprivation period, mice were injected intraperitoneally with 0.5ml of a solution containing the test substance. Thirty minutes after injection, individual mice were placed in one of eight 15 cm² test box with a stainless steel grid floor and a glass drinking tube which projected into the box. The drinking tube was connected to a reservoir containing a 20% sucrose solution, and the interior of the drinking tube contained an electrode enabling the detection of drinking contacts with the solution by measuring the flow of a weak (unnoticeable) electric current through mice by means of an electronic apparatus connected to the drinking tube electrode and the stainless steel grid floor. Consumption of the sucrose solution was measured over a 10 minute period by electronically recording the total amount of contact with the sucrose solution during the test session. The degree of appetite suppression produced by a given test substance was determined by statistical comparison of the duration of sucrose consumption by control (vehicle treated) mice with that of mice treated with a test substance. The degree of appetite suppression in a treated group of mice was expressed as percent of the control groups response.

### Example 6

### Test for appetite suppression in mice by fractions containing GLP-2.

Mice were tested for appetite suppression (see Example 5) after treatment with a test substance. The test substance consisted of extracts of the anorectic glucagonoma tumor prepared according to Example 3 (Gelfiltration fraction G4) or according to Example 4 (HPLC fraction G4H9) dissolved in phosphate buffered saline. The test solution containing lyophilized material from the Gelfiltration fraction G4 corresponding to 3.3 tumors suppressed sucrose consumption by 72%. Of the 10 HPLC sub-fractions of the G4 Gelfiltration fraction (see Example 4 and fig. 2), only the GLP-2 containing fraction, G4H9, gave a statistically significant suppression of appetite, suppressing sucrose consumption by 49%, when lyophilized material corresponding to 5.3 tumors was given.

### Example 7

### Test for appetite suppression in mice by synthetic GLP-2

Mice were tested for appetite suppression as described in Example 5 after treatment with a test substance consisting of synthetic porcine GLP-2 dissolved in phosphate buffered saline. Porcine GLP-2 has the following amino acid sequence: HADGSFSDEMNTVLDNLATRDFINWLLHTKITD. An intraperitoneal injection of test solution containing 50 micrograms of synthetic porcine GLP-2 suppressed sucrose consumption by 38%.

### Example 8

### Test method for measuring appetite suppression in mice.

The method is as in Example 5, but instead of 20% sucrose, a solution of infant formula milk (Complan ®) is used. The test substance is dissolved in a vehicle consisting of phosphate buffered saline with 1 % albumin. Test substances dissolved in vehicle are injected either intravenously (IV) in a volume of 100 microliters, or intra-cerebroventricularly (ICV) in a volume of 10 microliters.

### Example 9

### Test for appetite suppression in mice by synthetic GLP-2

Mice were tested for appetite suppression as described in Example 8 after treatment with a test substance consisting of synthetic human GLP-2. Human GLP-2 has the following amino acid sequence: H A D G S F S D E M N T I L D N L A A R D F I N W L I Q T K I T D. An IV injection of test solution containing 3 micrograms of synthetic human GLP-2 suppressed milk consumption by 24%, while ICV injections of 3 micrograms and 10 micrograms of synthetic human GLP-2 suppressed milk consumption by 32% and 35%, respectively.

## Claims

1. A peptide with the following amino acid sequence X1 H X2 D G S F S D E M N T X3 L D X4 L A X5 X6 D F I N W L X7 X8 T K I T D X9 wherein
X¹ is NH₂, DFPEEVAIVEELGRR, DFPEEVTIVEELGRR, DFPEEVNIVEELRRR, or a fragment thereof,
X² is Gly,
X³ is lie or Val,
X⁴ is Asn, Ser or His,
X⁵ is Ala or Thr,
X⁶ is Arg or Lys,
X⁷ is lie or Leu,
X⁸ is Gln or His, and
X⁹ is OH, Lys, Arg, Arg-Lys, Lys-Arg, Arg-Arg or Lys-Lys.

2. A peptide according to claim 1, wherein X¹ is NH₂.

3. A peptide according to claim 1, wherein X³ is IIe.

4. A peptide according to claim 1, wherein X⁴ is Asn.

5. A peptide according to claim 1, wherein X⁵ is Ala.

6. A peptide according to claim 1, wherein X⁶ is Arg.

7. A peptide according to claim 1, wherein X⁷ is Ile.

8. A peptide according to claim 1, wherein X⁸ is Gln.

9. A peptide according to claim 1, wherein X⁹ is OH.

10. A pharmaceutical composition comprising a peptide with the following amino acid sequence X¹ H X² D G S F S D E M N T X³ L D X⁴ L A X⁵ X⁶ D F I N W L X⁷ X⁸ T K I T D X⁹ wherein
X¹ is NH₂, DFPEEVAIVEELGRR, DFPEEVTIVEELGRR, DFPEEVNIVEELRRR, or a fragment thereof,
X² is Gly,
X³ is Ile or Val,
X⁴ is Asn, Ser or His,
X⁵ is Ala or Thr,
X⁶ is Arg or Lys,
X⁷ is lie or Leu,
X⁸ is Gln or His, and
X⁹ is OH, Lys, Arg, Arg-Lys, Lys-Arg, Arg-Arg or Lys-Lys
together with a pharmaceutically acceptable excipient or vehicle.

11. A pharmaceutical composition according to claim 10, wherein X¹ is NH₂.

12. A pharmaceutical composition according to claim 10, wherein X³ is Ile.

13. A pharmaceutical composition according to claim 10, wherein X⁴ is Asn.

14. A pharmaceutical composition according to claim 10, wherein X⁵ is Ala.

15. A pharmaceutical composition according to claim 10, wherein X⁶ is Arg.

16. A pharmaceutical composition according to claim 10, wherein X⁷ is Ile.

17. A pharmaceutical composition according to claim 10, wherein X⁸ is Gln.

18. A pharmaceutical composition according to claim 10, wherein X⁹ is OH.

19. A pharmaceutical composition comprising a peptide as defined in any one of the claims 10 to 18 in combination with another appetite-suppressing or satiety-inducing agent.

20. A pharmaceutical composition according to claim 19 wherein said other appetite-suppressing or satiety-inducing agent is glucagon-like peptide-1.

21. A pharmaceutical composition according to any one of the claims 10 to 20, wherein the amount of the peptide is in the range of from about 10 µg/kg body weight to about 5 mg/kg body weight.

22. Use of a peptide with the following amino acid sequence X1 H X2 D G S F S D E M N T X3 L D X4 L A X5 X6 D F I N W L X7 X8 T K I T D X9 wherein
X¹ is NH₂, DFPEEVAIVEELGRR, DFPEEVTIVEELGRR, DFPEEVNIVEELRRR, or a fragment thereof,
X² is Gly,
X³ is Ile or Val,
X⁴ is Asn, Ser or His,
X⁵ is Ala or Thr,
X⁶ is Arg or Lys,
X⁷ is lie or Leu,
X⁸ is Gln or His, and
X⁹ is OH, Lys, Arg, Arg-Lys, Lys-Arg, Arg-Arg or Lys-Lys
for the preparation of a pharmaceutical composition for appetite suppression or satiety induction.

23. The use according to claim 22, wherein X¹ is NH₂.

24. The use according to claim 22, wherein X³ is lle.

25. The use according to claim 22, wherein X⁴ is Asn.

26. The use according to claim 22, wherein X⁵ is Ala.

27. The use according to claim 22, wherein X⁶ is Arg.

28. The use according to claim 22, wherein X⁷ is lle.

29. The use according to claim 22, wherein X⁸ is Gln.

30. The use according to claim 22, wherein X⁹ is OH.

31. Use of a peptide as defined in any one of the claims 22 to 30 for the preparation of a pharmaceutical composition for the prophylaxis or treatment of diseases or disorders associated with impaired appetite regulation.

32. Use of a peptide as defined in any one of the claims 22 to 30 for the preparation of a pharmaceutical composition for the prophylaxis or treatment of obesity or type II diabetes.

33. Use of an HPLC fraction of a glucagonoma tumour extract prepared by acid ethanol extraction, gel filtration and preparative HPLC, said fraction being shown as fraction G4H9 in Fig. 2 and containing GLP-2 as a major component or comprising any single component of said fraction or a combination of two or more of the components of said fraction for the preparation of a pharmaceutical composition for appetite suppression or satiety induction.

34. Use of an HPLC fraction as defined in claim 33 for the preparation of a pharmaceutical composition for the prophylaxis or treatment of diseases or disorders associated with impaired appetite regulation.

35. Use of an HPLC fraction as defined in claim 33 for the preparation of a pharmaceutical composition for the prophylaxis or treatment of obesity or type II diabetes.

## Patentansprüche

1. Peptid mit der folgenden Aminosäuresequenz X1 H X2 D G S F S D E M N T X3 L D X4 L A X5 X6 D F I N W L X7 X8 T K I T D X9 wobei
X¹ NH₂, DFPEEVAIVEELGRR, DFPEEVTIVEELGRR,
DFPEEVNIVEELRRR oder ein Fragment davon ist,
X² ist Gly
X³ ist Ile oder Val,
X⁴ ist Asn, Ser oder His,
X⁵ ist Ala oder Thr,
X⁶ ist Arg oder Lys,
X⁷ ist Ile oder Leu,
X⁸ ist Gln oder His und
X⁹ ist OH, Lys, Arg, Arg-Lys, Lys-Arg, Arg-Arg oder Lys-Lys.

2. Peptid gemäß Anspruch 1, wobei X¹ NH₂ ist.

3. Peptid gemäß Anspruch 1, wobei X³ Ile ist.

4. Peptid gemäß Anspruch 1, wobei X⁴ Asn ist.

5. Peptid gemäß Anspruch 1, wobei X⁵ Ala ist.

6. Peptid gemäß Anspruch 1, wobei X⁶ Arg ist.

7. Peptid gemäß Anspruch 1, wobei X⁷ Ile ist.

8. Peptid gemäß Anspruch 1, wobei X⁸ Gln ist.

9. Peptid gemäß Anspruch 1, wobei X⁹ OH ist.

10. Pharmazeutische Zusammensetzung umfassend ein Peptid mit der folgenden Aminosäuresequenz X¹ H X² D G S F S D E M N T X³ L D X⁴ L A X⁵ X⁶ D F I N W L X⁷ X⁸ T K I T D X⁹ wobei
X¹ NH₂, DFPEEVAIVEELGRR, DFPEEVTIVEELGRR, DFPEEVNIVEELRRR oder ein Fragment davon ist,
X² ist Gly
X³ ist Ile oder Val,
X⁴ ist Asn, Ser oder His,
X⁵ ist Ala oder Thr,
X⁶ ist Arg oder Lys,
X⁷ ist Ile oder Leu,
X⁸ ist Gln oder His und
X⁹ ist OH, Lys, Arg, Arg-Lys, Lys-Arg, Arg-Arg oder Lys-Lys
zusammen mit einem pharmazeutisch verträglichen Arzneimittelträger oder Vehikel.

11. Pharmazeutische Zusammensetzung nach Anspruch 10, wobei X¹ NH₂ ist.

12. Pharmazeutische Zusammensetzung nach Anspruch 10, wobei X³ Ile ist.

13. Pharmazeutische Zusammensetzung nach Anspruch 10, wobei X⁴ Asn ist.

14. Pharmazeutische Zusammensetzung nach Anspruch 10, wobei X⁵ Ala ist.

15. Pharmazeutische Zusammensetzung nach Anspruch 10, wobei X⁶ Arg ist.

16. Pharmazeutische Zusammensetzung nach Anspruch 10, wobei X⁷ Ile ist.

17. Pharmazeutische Zusammensetzung nach Anspruch 10, wobei X⁸ Gln ist.

18. Pharmazeutische Zusammensetzung nach Anspruch 10, wobei X⁹ OH ist.

19. Pharmazeutische Zusammensetzung umfassend ein wie in einem beliebigen der Ansprüche 10-18 definiertes Peptid in Kombination mit einem anderen appetitzügelnden oder Sattheit induzierenden Mittel.

20. Pharmazeutische Zusammensetzung nach Anspruch 19, wobei das genannte appetitzügelnde oder Sattheit induzierende Mittel Glucagon-artiges Peptid-1 ist.

21. Pharmazeutische Zusammensetzung nach einem beliebigen der Ansprüche 10 bis 20, wobei die Menge des Peptids in dem Bereich von etwa 10 µg/kg Körpergewicht bis etwa 5 mg/kg Körpergewicht liegt.

22. Verwendung eines Peptids mit der folgenden Aminosäuresequenz X¹ H X² D G S F S D E M N T X³ L D X⁴ L A X⁵ X⁶ D F I N W L X⁷ X⁸ T K I T D X⁹ wobei
X¹ NH₂, DFPEEVAIVEELGRR, DFPEEVTIVEELGRR,
DFPEEVNIVEELRRR oder ein Fragment davon ist,
X² ist Gly
X³ ist Ile oder Val,
X⁴ ist Asn, Ser oder His,
X⁵ ist Ala oder Thr,
X⁶ ist Arg oder Lys,
X⁷ ist Ile oder Leu,
X⁸ ist Gln oder His und
X⁹ ist OH, Lys, Arg, Arg-Lys, Lys-Arg, Arg-Arg oder Lys-Lys
zur Zubereitung einer pharmazeutischen Zusammensetzung zur Appetitzügelung oder Sättigkeitsinduktion.

23. Verwendung nach Anspruch 22, wobei X¹ NH₂ ist.

24. Verwendung nach Anspruch 22, wobei X³ Ile ist.

25. Verwendung nach Anspruch 22, wobei X⁴ Asn ist.

26. Verwendung nach Anspruch 22, wobei X⁵ Ala ist.

27. Verwendung nach Anspruch 22, wobei X⁶ Arg ist.

28. Verwendung nach Anspruch 22, wobei X⁷ Ile ist.

29. Verwendung nach Anspruch 22, wobei X⁸ Gln ist.

30. Verwendung nach Anspruch 22, wobei X⁹ OH ist.

31. Verwendung eines wie in einem beliebigen der Ansprüche 22 bis 30 definierten Peptids zur Zubereitung einer pharmazeutischen Zusammensetzung zur Prophylaxe oder Behandlung von mit gestörter Appetitregulation assoziierten Erkrankungen und Funktionsstörungen.

32. Verwendung eines wie in einem beliebigen der Ansprüche 22 bis 30 definierten Peptids zur Zubereitung einer pharmazeutischen Zusammensetzung zur Prophylaxe oder Behandlung von Fettleibigkeit oder Typ-II-Diabetes.

33. Verwendung einer HPLC-Fraktion eines Glucagonomatumor-Extrakts hergestellt mittels saurer Ethanolextraktion, Gelfiltration und präparativer HPLC, wobei die genannte Fraktion als Fraktion G4H9 in Figur 2 gezeigt ist und GLP-2 als Hauptkomponente enthält oder eine beliebige Einzelkomponente der genannten Fraktion oder eine Kombination von zwei oder mehreren der Komponenten der genannten Fraktion für die Zubereitung einer pharmazeutischen Zusammensetzung zur Appetitzügelung oder Sättigkeitsinduktion umfasst.

34. Verwendung einer wie in Anspruch 33 definierten HPLC-Fraktion für die Zubereitung einer pharmazeutischen Zusammensetzung zur Prophylaxe oder Behandlung von mit gestörter Appetitregulation assoziierten Erkrankungen oder Funktionsstörungen.

35. Verwendung einer wie in Anspruch 33 definierten HPLC-Fraktion für die Zubereitung einer pharmazeutischen Zusammensetzung zur Prophylaxe oder Behandlung von Fettleibigkeit oder Typ-II-Diabetes.

## Revendications

1. Peptide ayant la séquence suivante d'acides aminés X¹HX²DGSFSDEMNTX³LDX⁴LAX⁵X⁶DFINWLX⁷X⁸TKITDX⁹ dans laquelle
X¹ est NH₂, DFPEEVAIVEELGRR, DFPEEVTIVEELGRR, DFPEEVNIVEELGRR,
ou un fragment de ceux-ci,
X² est Gly,
X³ est Ile ou Val,
X⁴ est Asn, Ser ou His,
X⁵ est Ala ou Thr,
X⁶ est Arg ou Lys,
X⁷ est Ile ou Leu,
X⁸ est Gln ou His, et
X⁹ est OH, Lys, Arg, Arg-Lys, Lys-Arg, Arg-Arg ou Lys-Lys.

2. Peptide selon la revendication 1, dans lequel X¹ est NH₂.

3. Peptide selon la revendication 1, dans lequel X³ est Ile.

4. Peptide selon la revendication 1, dans lequel X⁴ est Asn.

5. Peptide selon la revendication 1, dans lequel X⁵ est Ala.

6. Peptide selon la revendication 1, dans lequel X⁶ est Arg.

7. Peptide selon la revendication 1, dans lequel X⁷ est Ile.

8. Peptide selon la revendication 1, dans lequel X⁸ est Gln.

9. Peptide selon la revendication 1, dans lequel X⁹ est OH.

10. Composition pharmaceutique comprenant un peptide ayant la séquence suivante d'acides aminés X^1HX^2DGSFSDEMNTX^3LDX^4LAX^5X^6DFINWLX^7X^8TKITDX^9 dans laquelle
X¹ est NH₂, DFPEEVAIVEELGRR, DFPEEVTIVEELGRR, DFPEEVNIVEELGRR,
ou un fragment de ceux-ci,
X² est Gly,
X³ est Ile ou Val,
X⁴ est Asn, Ser ou His,
X⁵ est Ala ou Thr,
X⁶ est Arg ou Lys,
X⁷ est Ile ou Leu,
X⁸ est Gln ou His, et
X⁹ est OH, Lys, Arg, Arg-Lys, Lys-Arg, Arg-Arg ou Lys-Lys,
en même temps qu'un excipient ou un véhicule acceptable d'un point de vue pharmaceutique.

11. Composition pharmaceutique selon la revendication 10, dans laquelle X¹ est NH₂.

12. Composition pharmaceutique selon la revendication 10, dans laquelle X³ est Ile.

13. Composition pharmaceutique selon la revendication 10, dans laquelle X⁴ est Asn.

14. Composition pharmaceutique selon la revendication 10, dans laquelle X⁵ est Ala.

15. Composition pharmaceutique selon la revendication 10, dans laquelle X⁶ est Arg.

16. Composition pharmaceutique selon la revendication 10, dans laquelle X⁷ est Ile.

17. Composition pharmaceutique selon la revendication 10, dans laquelle X⁸ est Gln.

18. Composition pharmaceutique selon la revendication 10, dans laquelle X⁹ est OH.

19. Composition pharmaceutique comprenant un peptide selon l'une quelconque des revendications 10 à 18 en combinaison avec un autre agent suppresseur d'appétit ou inducteur de satiété.

20. Composition pharmaceutique selon la revendication 19, dans laquelle ledit autre agent suppresseur d'appétit ou inducteur de satiété est un peptide-1 analogue au glucagon.

21. Composition pharmaceutique selon l'une quelconque des revendications 10 à 20, dans laquelle la quantité du peptide est comprise dans la plage d'environ 10 µg/kg de poids corporel à environ 5 mg/kg de poids corporel.

22. Utilisation d'un peptide ayant la séquence suivante d'acides aminés X¹HX²DGSFSDEMNTX³LDX⁴LAX⁵X⁶DFINWLX⁷X⁸TKITDX⁹ dans laquelle
X¹ est NH₂, DFPEEVAIVEELGRR, DFPEEVTIVEELGRR, DFPEEVNIVEELGRR,
ou un fragment de ceux-ci,
X² est Gly,
X³ est Ile ou Val,
X⁴ est Asn, Ser ou His,
X⁵ est Ala ou Thr,
X⁶ est Arg ou Lys,
X⁷ est Ile ou Leu,
X⁸ est Gln ou His, et
X⁹ est OH, Lys, Arg, Arg-Lys, Lys-Arg, Arg-Arg ou Lys-Lys,
pour préparer une composition pharmaceutique destinée à la suppression de l'appétit ou à l'induction de la satiété.

23. Utilisation selon la revendication 22, dans laquelle X¹ est NH₂.

24. Utilisation selon la revendication 22, dans laquelle X³ est Ile.

25. Utilisation selon la revendication 22, dans laquelle X⁴ est Asn.

26. Utilisation selon la revendication 22, dans laquelle X⁵ est Ala.

27. Utilisation selon la revendication 22, dans laquelle X⁶ est Arg.

28. Utilisation selon la revendication 22, dans laquelle X⁷ est Ile.

29. Utilisation selon la revendication 22, dans laquelle X⁸ est Gln.

30. Utilisation selon la revendication 22, dans laquelle X⁹ est OH.

31. Utilisation d'un peptide selon l'une quelconque des revendications 22 à 30 pour la préparation d'une composition pharmaceutique destinée à la prophylaxie ou au traitement de maladies ou de troubles associés à une dégradation de la régulation de l'appétit.

32. Utilisation d'un peptide selon l'une quelconque des revendications 22 à 30, pour la préparation d'une composition pharmaceutique destinée à la prophylaxie et au traitement de l'obésité ou du diabète de type II.

33. Utilisation d'une fraction CLHP d'un extrait de tumeur de glucagonome, préparé par extraction acide-éthanol, filtration sur gel et CLHP préparative, ladite fraction étant représentée par la fraction G4H9 sur la Figure 2 et contenant du GLP-2 en tant que composant principal ou comprenant un composant unique quelconque de ladite fraction ou une combinaison d'au moins deux des composants de ladite fraction, pour la préparation d'une composition pharmaceutique destinée à la suppression de l'appétit ou à l'induction de la satiété.

34. Utilisation d'une fraction CLHP selon la revendication 33 pour la préparation d'une composition pharmaceutique destinée à la prophylaxie et au traitement de maladies ou de troubles associés à une dégradation de la régulation de l'appétit.

35. Utilisation d'une fraction CLHP selon la revendication 33 pour la préparation d'une composition pharmaceutique destinée à la prophylaxie ou au traitement de l'obésité ou du diabète de type II.
